# NOUVEAU FASCICULE DE BREVET EUROPEEN

(11) **EP 0 629 396 B2**
(45) Date de publication et mention de la décision concernant l'opposition: **24.01.2001**
(45) Mention de la délivrance du brevet: 23.07.1997
(21) Numéro de dépôt: 94401294.7
(22) Date de dépôt: 09.06.1994
(51) Int. Cl.: A61K 7/48, A61K 7/50, A61K 7/00

(54) **Emulsion cosmétique ou dermatologique huile-dans-eau contenant une composition auto-émulsionnable**
Öl-in-Wasser kosmetische oder dermatologische Emulsion, die eine selbstemulgierende Zusammensetzung enthält
Oil-in-water cosmetic or dermatological emulsion containing a self-emulsifiable composition

(30) Priorité: 10.06.1993 FR 9307004
(43) Date de publication de la demande: 21.12.1994
(73) Titulaire: L'OREAL, 75008 Paris (FR)
(72) Inventeur: Picard, Elisabeth, 92360 Meudon La Foret (FR); Lambert, Jacqueline, F-75014 Paris (FR); Griat, Jacqueline, F-94480 Ablon (FR)
(74) Mandataire: Stalla-Bourdillon, Bernard

(56) Documents cités:
- EP-A- 0 006 741
- EP-B- 0 553 241
- EP-B- 0 554 292
- WO-A-91/15184
- WO-A-92/06778
- FR-A- 9 307 004
- Cetearyl Glucoside, New approach to sun care emulsions, C. Amalric et al, DCI, March 1994

## Description

La présente invention a pour objet une composition sous forme d'une émulsion huile-dans-eau (H/E) stable, fine et homogène, essentiellement constituée d'une phase huileuse pouvant contenir en une proportion inférieure à 15 % en poids au moins une huile à teneur élevée en triglycérides d'acide linoléique, d'une composition auto-émulsionnable et d'un co-émulsionnant, et son utilisation dans différentes applications cosmétiques ou dermatologiques.

On sait que les émulsions utilisées dans les domaines cosmétique et dermatologique comprennent généralement, pour faciliter leur émulsification, des tensioactifs de nature non-ionique, de type hydrophile, obtenus par greffage d'une chaîne polyoxyéthylée. En vue d'éviter l'emploi de tels tensioactifs plus ou moins irritants en raison d'éventuelles impuretés dues à leurs procédés de préparation, il a été proposé dans la demande WO 92/06778, l'utilisation d'une composition auto-émulsionnable à base d'alcools gras et d'alkylpolyoside.

Toutefois, les émulsions obtenues par l'utilisation d'une telle composition auto-émulsionnable ne sont pas totalement satisfaisantes.

En effet, elles manquent de finesse et d'homogénéité et sont notamment épaisses. En outre, elles pénètrent difficilement par application sur la peau, ce qui les rend cosmétiquement peu acceptables.

Il a maintenant été constaté de façon surprenante et inattendue qu'en associant à la composition auto-émulsionnable selon WO 92/06778, un co-émulsionnant résultant d'une sélection bien particulière, il était possible de résoudre les inconvénients précités.

Les émulsions (H/E) selon l'invention sont en effet très stables, légères et homogènes ce qui correspond aux critères recherchés en cosmétique et en dermatologie notamment lorsque les émulsions sont destinées aux soins du visage ou du corps.

La présente invention a donc pour objet une composition cosmétique ou dermatologique sous forme d'une émulsion huile-dans-eau contenant :
(a) de 5 à 40 % en poids d'une phase huileuse pouvant contenir, en une proportion inférieure à 15 % en poids, au moins une huile végétale ayant plus de 40 % en poids de triglycérides d'acide linoléique,
(b) de 0,5 à 5 % en poids d'une composition ou association auto-émulsionnable comprenant de 60 à 90 % en poids d'au moins un alcool gras ayant de 12 à 22 atomes de carbone, de 10 à 40 % en poids d'un alkylpolyoside dont la chaîne alkyle a de 12 à 22 atomes de carbone et de 0 à 5 % en poids de polyoside,
(c) de 0,5 à 4 % en poids d'au moins un co-émulsionnant choisi parmi les acides gras ayant de 14 à 22 atomes de carbone, les mélanges desdits acides gras et d'alcools gras ayant de 14 à 22 atomes de carbone, les alkyléthers de glycéryle dont la chaîne alkyle a de 14 à 22 atomes de carbone et les composés de formule : 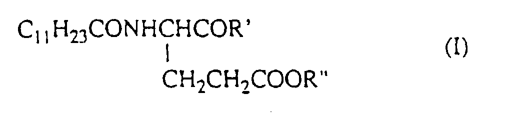
dans laquelle :
R' et R", identiques ou différents, représentent un radical cholestéryle, béhényle ou 2-octyldodécyle, sous réserve que lorsqu'un seul co-émulsionnant est présent, sa proportion en poids est égale ou inférieure à 2 %, le reste étant essentiellement constitué par une phase aqueuse.

Selon un mode de réalisation préféré, le rapport pondéral entre la phase huileuse et l'alkylpolyoside de l'association auto-émulsionnable est égal ou supérieur à environ 15.

Parmi les huiles de la phase huileuse on peut citer :
- les huiles minérales telles que l'huile de paraffine, l'huile de vaseline et les huiles minérales ayant un point d'ébullition compris entre 300 et 400°C ;
- les huiles d'origine animale telles que le perhydrosqualène;
- les huiles d'origine végétale constituées de moins de 40 % en poids de triglycérides d'acide linoléique telles que l'huile d'amande douce, l'huile d'avocat, l'huile de ricin, l'huile d'olive, l'huile de jojoba, l'huile d'arachide, l'huile de colza, l'huile de coprah, l'huile de noisette, le beurre de karité, l'huile de palme, l'huile de noyau d'abricot et l'huile de calophyllum ;
- les huiles synthétiques telles que l'huile de purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le dicaprylate de propylène glycol et les esters dérivés d'acide lanolique tels que le lanolate d'isopropyle, le lanolate d'isocétyle et les isoparaffines.

Parmi les huiles utilisables selon l'invention, on peut également citer : les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés en C₁₀-C₁₈, les huiles de silicone volatiles ou non, et les huiles fluorées et perfluorées.

Parmi les huiles végétales ayant plus de 40 % en poids de triglycérides d'acide linoléique, on peut citer : l'huile de germes de maïs, l'huile de germes de blé, l'huile de soja, l'huile de tournesol, l'huile de sésame, l'huile de pépins de raisin, l'huile d'onagre, l'huile de carthame, l'huile de passiflore et l'huile de seigle.

Parmi les associations ou compositions auto-émulsionnables décrites dans WO 92/06778, on peut notamment citer le produit commercialisé sous la dénomination de "Montanov 68" par la Société Seppic qui est essentiellement constitué par l'association d'environ 77 % d'alcool cétylstéarylique (C₁₆-C₁₈) et d'environ 23 % de cétéarylglucosides.

Selon un mode de réalisation particulier de l'invention, le radical alkyle de l'alkylpolyoside est identique à celui de l'alcool gras de la composition auto-émulsionnable.

Selon un mode de réalisation préféré des compositions selon l'invention, le rapport pondéral entre la phase huileuse et la composition auto-émulsionnable est compris entre 6 et 30.

Parmi les co-émulsionnants utilisables dans les compositions selon l'invention, on peut citer parmi les alcools gras ayant de 14 à 22 atomes de carbone, l'alcool stéarylique et l'alcool cétylique et leurs mélanges, parmi les acides gras ayant de 14 à 22 atomes de carbone, l'acide stéarique, et parmi les alkyléthers de glycéryle ayant une chaîne alkyle ayant de 14 à 22 atomes de carbone, l'alcool chimylique et l'alcool batylique.

Parmi les composés de formule (I) utilisables comme co-émulsionnants, on peut citer le produit commercialisé sous la dénomination de "_{ELDEW-CL-301}" par la Société Ajinomoto.

Comme co-émulsionnant appartenant au groupe des acides gras ayant de 14 à 22 atomes de carbone, on peut utiliser selon l'invention un produit d'origine naturelle à savoir la cire de sumac qui contient environ 40 % d'acides gras en C₁₆ et 50 % d'acides gras en C₁₈.

Les compositions selon l'invention peuvent contenir en outre divers adjuvants. Parmi les adjuvants de la phase grasse, on peut citer notamment des cdorants, des principes actifs lipophiles, des filtres solaires, des antioxydants et des conservateurs.

Parmi les adjuvants de la phase aqueuse, on peut citer notamment des dérivés hydrosolubles tels que des colorants et des conservateurs, des principes actifs tels que l'hyaluronate de sodium et le gluconate de magnésium, des oligo-éléments, des dérivés biologiques tels que l'urée, l'acide lactique, l'acide pyrrolidone carboxylique et ses sels notamment son sel de sodium, des polyols tels que le propylène glycol, le butylène 1-3 glycol, le glycérol, le polyglycérol, le sorbitol, le glucose et le saccharose, des sels tels que le sulfate de magnésium et le chlorure de sodium, des minéraux argileux gonflant en milieu aqueux tels que la saponite, l'hectorite et la smectite, des acides aminés, des agents gélifiants aqueux comme des polysaccharides tels que les dérivés cellulosiques (carboxyméthylcellulose, hydroxypropylméthylcellulose,...), des gommes telles que la gomme de xanthane et la gomme de caroube, des protéines telles que les kératines sulfoniques, le collagène et l'élastine, des silicates tels que le silicate d'aluminium et le silicate de magnésium, des dérivés acryliques tels que les Carbomers, les polyacrylates ou polyméthacrylates de glycéryle et les polyacrylamides.

Les compositions selon l'invention peuvent également contenir divers ingrédients cosmétiques tels que des huiles essentielles, des parfums, des pigments, des charges, des vitamines et des céramides.

Les compositions selon l'invention sous forme d'émulsions (H/E) peuvent être utilisées dans différentes applications cosmétiques ou dermatologiques, par exemple sous forme de crèmes pour le visage, pour le corps, pour le cuir chevelu ou pour la chevelure, ou sous forme de laits pour le corps ou pour le démaquillage.

Ces compositions peuvent être également utilisées pour le maquillage, notamment sous forme de fonds de teint, après addition de pigments. Elles peuvent être utilisées également comme crèmes solaires après addition de filtres UV-B et/ou UV-A ou comme crèmes ou laits après-solaires après addition de composés apaisants tels que le panthénol.

Les émulsions (H/E) selon l'invention sont obtenues selon les méthodes classiques par mélange à chaud, à une température d'environ 70°C, de la phase grasse comprenant la phase huileuse et les émulsionnants et autres additifs à la phase aqueuse sous forte agitation, puis addition de la quantité d'eau complémentaire et refroidissement.

On va maintenant donner à titre d'illustration plusieurs exemples de compositions selon l'invention sous forme d'émulsions huile-dans-eau.

### EXEMPLES DE COMPOSITIONS

### EXEMPLE 1 :Crème de soin

| | |
|---|---|
| - Huile de sésame | 10 % |
| - Polyisobutène hydrogéné | 15 % |
| - Composition auto-émulsionnable commercialisée sous la dénomination de "Montanov 68" par la Société Seppic | 3 % |
| - Alcool stéarylique | 2 % |
| - Acide stéarique | 1 % |
| - Conservateurs | 0,2 % |
| - Parfum | 0,1 % |
| - Hydroxyde de sodium | 0,012 % |
| - Glycérine | 3 % |
| - Eau q.s.p. | 100 % |

La crème obtenue est stable, fine et homogène. Elle est douce et pénètre bien.

### EXEMPLE 2 : Crème de soin pour peau grasse

| | |
|---|---|
| - Cyclométhicone | 10 % |
| - Composition auto-émulsionnable commercialisée sous la dénomination de "Montanov 68" par la Société Seppic | 1,5 % |
| - Alcool stéarylique | 1% |
| - Acide stéarique | 0,5 % |
| - Conservateurs | 0,2 % |
| - Hydroxyde de sodium | 0,006 % |
| - Gomme de xanthane | 0,2 % |
| - Propylène glycol | 3 % |
| - Eau q.s.p. | 100 % |

La crème obtenue est légère, douce, fraîche à l'application et pénètre bien sans effet gras sur la peau.

### EXEMPLE 3 : Fluide anti-âge

| | |
|---|---|
| - Huile de noyau d'abricot | 25 % |
| - Composition auto-émulsionnable commercialisée sous la dénomination de "Montanov 68" par la Société Seppic | 3 % |
| - Co-émulsionnant commercialisé sous la dénomination de "_{ELDEW CL-301}" par la Société Ajinomot | 2 % |
| - Méthoxycinnamate d'octyle | 1 % |
| - Mélange de dioxyde de titane et de stéarate d'aluminium commercialisé sous la dénomination de "Micro-titanium dioxide-_{MT 100T}" par la Société Tayca | 1 % |
| - Conservateurs | 0,2 % |
| - Miel | 1 % |
| - Protéine de soja hydrolysée | 0,1 % |
| - Glycérine | 3 % |
| - Eau q.s.p. | 100 % |

La crème obtenue est fluide, très douce et pénètre bien. Elle a un bon effet protecteur.

### EXEMPLE 4 : Crème de soin

| | |
|---|---|
| - Cyclométhicone | 10 % |
| - Mélange constitué de polyacrylamide-isoparaffine en C₁₃-C₁₄-Laureth 7 | 0,8 % |
| - Composition auto-émulsionnable commercialisée sous la dénomination de "Montanov 68" par la Société Seppic | 2 % |
| - Alcool stéarylique | 1 % |
| - Acide stéarique | 0,5 % |
| - Conservateurs | 0,65 % |
| - Lysine | 0,025 % |
| - Sel disodique de l'acide éthylènediaminetétraacétique | 0,05% |
| - Gomme de xanthane | 0,2 % |
| - Glycérine | 3 % |
| - Eau q.s.p. | 100 % |

La crème obtenue est fine, douce et pénètre bien.

### EXEMPLE 5 : Crème de soin

| | |
|---|---|
| - Cyclométhicone | 10 % |
| - Mélange constitué de polyacrylamide-isoparaffine en C₁₃-C₁₄-Laureth 7 | 0,8 % |
| - Perfluoropolyméthylisopropyléther | 0,5 % |
| - Composition auto-émulsionnable commercialisée sous la dénomination de "Montanov 68" par la Société Seppic | 2 % |
| - Alcool stéarylique | 1% |
| - Acide stéarique | 0,5 % |
| - Conservateurs | 0,65 % |
| - Lysine | 0,025 % |
| - Sel disodique de l'acide éthylènediaminetétraacétique | 0,05 % |
| - Gomme de xanthane | 0,2 % |
| - Glycérine | 3 % |
| - Eau q.s.p. | 100 % |

La crème obtenue est stable, fine et homogène.

### ETUDE COMPARATIVE

On a comparé, sur le plan de la stabilité et de la finesse, l'émulsion (H/E) selon l'exemple 2 ci-dessus à cette même émulsion (HIE) mais ne contenant pas de co-émulsionnant, cette dernière ayant la composition suivante :

| | |
|---|---|
| - Cyclométhicone | 10 % |
| - Composition auto-émulsionnable commercialisée sous la dénomination de "Montanov 68" par la Société Seppic | 1,5 % |
| - Conservateurs | 0,2 % |
| - Hydroxyde de sodium | 0,006 % |
| - Gomme de xanthane | 0,2 % |
| - Propylène glycol | 3 % |
| - Eau q.s.p. | 100 % |

Après avoir laissé reposer les émulsions obtenues pendant une heure à une température de 20°C, on a procédé à leur examen à l'aide d'un microscope ayant un grossissement de 100.

Des photos des émulsions ont alors été prises et celles-ci sont annexées sous les références Photo (A) et Photo (B).

La Photo (A) correspond à l'émulsion de l'exemple 2 et la Photo (B) à celle de l'exemple comparatif.

Comme on peut le constater, l'émulsion de la Photo (A) est beaucoup plus fine et homogène que celle de la Photo (B) qui laisse apparaître des globules dénotant un manque d'homogénéité et de stabilité.

## Revendications

1. Composition cosmétique ou dermatologique sous forme d'une émulsion huile-dans-l'eau stable, caractérisée par le fait qu'elle contient :
(a) de 5 à 40 % en poids d'une phase huileuse pouvant contenir, en une proportion inférieure à 15 % en poids, au moins une huile végétale ayant plus de 40 % en poids de triglycérides d'acide linoléique,
(b) de 0,5 à 5 % en poids d'une composition auto-émulsionnable comprenant de 60 à 90 % en poids d'au moins un alcool gras ayant de 12 à 22 atomes de carbone, de 10 à 40 % en poids d'un alkylpolyoside dont la chaîne alkyle a de 12 à 22 atomes de carbone et de 0 à 5 % en poids de polyoside,
(c) de 0,5 à 4 % en poids d'au moins un co-émulsionnant choisi parmi les acides gras ayant de 14 à 22 atomes de carbone, les mélanges desdits acides gras et d'alcools gras ayant de 14 à 22 atomes de carbone, les alkyléthers de glycéryle dont la chaîne alkyle a de 14 à 22 atomes de carbone, et les composés de formule : 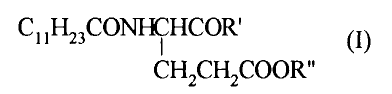 dans laquelle :
R' et R", identiques ou différents, représentent un radical cholestéryle, béhényle ou 2-octyldodécyle, sous réserve que lorsqu'un seul co-émulsionnant est présent, sa proportion en poids est égale ou inférieure à 2 %, et
(d) le reste étant essentiellement constitué par une phase aqueuse.

2. Composition selon la revendication 1, caractérisée par le fait que le rapport pondéral entre la phase huileuse et l'alkylpolyoside de la composition auto-émulsionnable est égal ou supérieur à environ 15.

3. Composition selon la revendication 2, caractérisée par le fait que l'alcool gras ayant de 12 à 22 atomes de carbone de la composition auto-émulsionnable est l'alcool cétylstéarylique (C₁₆-C₁₈) et l'alkylpolyoside est du cétéarylglucoside.

4. Composition selon la revendication 1, caractérisée par le fait que le co-émulsionnant est choisi dans le groupe constitué par : l'acide stéarique, l'alcool chimylique, l'alcool batylique et leurs mélanges ainsi que le mélange de l'acide stéarique et de l'alcool stéarylique.

5. Composition selon l'une quelconque des revendications précédentes, caractérisée par le fait que la phase grasse de ladite émulsion contient en outre un adjuvant choisi parmi les colorants, les principes actifs lipophiles, les filtres solaires, les antioxydants et les conservateurs.

6. Composition selon l'une quelconque des revendications précédentes, caractérisée par le fait que la phase aqueuse de ladite émulsion contient en outre un adjuvant choisi parmi les colorants, les conservateurs, les principes actifs hydrophiles, les oligo-éléments, les dérivés biologiques, les polyols, les sels, les minéraux argileux gonflant en milieu aqueux, les acides aminés et les agents gélifiants aqueux.

7. Composition selon l'une quelconque des revendications précédentes, caractérisée par le fait qu'elle contient en outre au moins une substance active choisie parmi les huiles essentielles, les parfums, les pigments, les charges, les vitamines et les céramides.

## Claims

1. Cosmetic or dermatological composition in the form of a stable oil-in-water emulsion, characterized in that it contains:
(a) from 5 to 40 % % by weight of an oily phase which may contain, in a proportion of less than 15 % by weight, at least one vegetable oil which has more than 40 % by weight of linoleic acid triglycerides,
(b) from 0.5 to 5 % by weight of an auto-emulsifiable composition containing from 60 to 90 % by weight of at least one fatty alcohol having from 12 to 22 carbon atoms, from 10 to 40 % by weight of an alkyl polyoside, the alkyl chain of which has from 12 to 22 carbon atoms and from 0 to 5 % by weight of polyoside,
(c) from 0.5 to 4 % by weight of at least one co-emulsifying agent chosen from fatty acids having from 14 to 22 carbon atoms, the mixtures of the said fatty acids and fatty alcohols having from 14 to 22 carbon atoms, alkyl glyceryl ethers, the alkyl chain of which has from 14 to 22 carbon atoms, the compounds of formula: 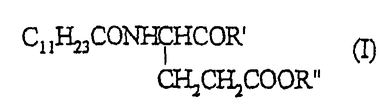 in which:
R' and R", which may be identical or different, represent a cholesteryl, behenyl or 2-octyldodecyl radical, with the proviso that when a lone co-emulsifying agent is present, its proportion by weight is equal to or less than 2 %, and
(d) the remainder essentially consisting of an aqueous phase.

2. Composition according to Claim 1, characterized in that the weight ratio between the oily phase and the alkyl polyoside of the auto-emulsifiable composition is greater than or equal to approximately 15.

3. Composition according to Claim 2, characterized in that the fatty alcohol of the auto-emulsifiable composition having from 12 to 22 carbon atoms is (C₁₆-C₁₈) cetyl/stearyl alcohol and the alkyl polyoside is cetearyl glucoside.

4. Composition according to Claim 1, characterized in that the co-emulsifying agent is chosen from the group consisting of: stearic acid, chimyl alcohol, batyl alcohol and their mixtures as well as the mixture of stearic acid and stearyl alcohol.

5. Composition according to any one of the preceding claims, characterized in that the fatty phase of the said emulsion additionally contains an adjuvant chosen from dyes, lipophilic active principles, sunscreen agents, antioxidants and preservatives.

6. Composition according to any one of the preceding claims, characterized in that the aqueous phase of the said emulsion additionally contains an adjuvant chosen from dyes, preservatives, hydrophilic active principles, trace elements, biological derivatives, polyols, salts, clayey minerals which swell in aqueous medium, amino acids and aqueous gelling agents.

7. Composition according to any one of the preceding claims, characterized in that it additionally contains at least one active substance chosen from essential oils, perfumes, pigments, fillers, vitamins and ceramides.

## Patentansprüche

1. Kosmetische oder dermatologische Zubereitung in Form einer stabilen Öl-in-Wasser-Emulsion, dadurch gekennzeichnet, daß sie die folgenden Bestandteile enthält:
(a) 5 bis 40 Gew.-% einer Ölphase, die in einem Mengenverhältnis von weniger als 15 Gew.-% mindestens ein pflanzliches Öl mit mehr als 40 Gew.-% Linolsäuretriglyceriden enthalten kann,
(b) 0,5 bis 5 Gew.-% einer selbstemulgierbaren Zusammensetzung, die 60 bis 90 Gew.-% mindestens eines Fettalkohols mit 12 bis 22 Kohlenstoffatomen, 10 bis 40 Gew.-% Alkylpolyosid, dessen Alkylkette 12 bis 22 Kohlenstoffatome hat, sowie 0 bis 5 Gew.-% Polyosid umfaßt,
(c) 0,5 bis 4 Gew.-% mindestens eines Coemulgators, ausgewählt unter den Fettsäuren mit 14 bis 22 Kohlenstoffatomen, den Gemischen der Fettsäuren und Fettalkohole mit 14 bis 22 Kohlenstoffatomen, Glycerylalkylethern, deren Alkylkette 14 bis 22 Kohlenstoffatome aufweist, sowie Verbindungen der Formel 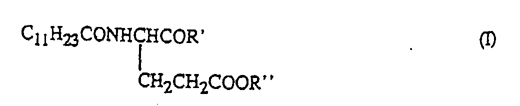 worin
R' und R", die gleich oder verschieden sind, einen Cholesteryl-, Behenyl- oder 2-Oktyldodecylrest bedeuten, unter dem Vorbehalt, daß im Falle der Anwesenheit nur eines einzigen Coemulgators dessen Gewichtsanteil 2 % oder weniger beträgt, und
(d) der restliche Anteil im wesentlichen aus einer wäßrigen Phase besteht.

2. Zubereitung nach Anspruch 1, dadurch gekennzeichnet, daß das Gewichtsverhältnis zwischen der Ölphase und dem Alkylpolyosid der selbstemulgierbaren Zusammensetzung gleich 15 ist oder mehr als etwa 15 beträgt.

3. Zubereitung nach Anspruch 2, dadurch gekennzeichnet, daß der Fettalkohol mit 12 bis 22 Kohlenstoffatomen der selbstemulgierbaren Zusammensetzung einen C₁₆-C₁₈-Cetylstearylalkohol darstellt und das Alkylpolyosid Cetearylglykosid ist.

4. Zubereitung nach Anspruch 1, dadurch gekennzeichnet, daß der Coemulgator aus der aus Stearinsäure, Chimylalkohol, Batylalkohol und deren Gemischen sowie dem Gemisch aus Stearinsäure und Stearylalkohol bestehenden Gruppe ausgewählt ist.

5. Zubereitung nach einem der vorstehenden Ansprüche, dadurch gekennzeichnet, daß die Fettphase der Emulsion zusätzlich noch einen Hilfsstoff enthält, der aus Farbstoffen, lipophilen Wirkstoffen, Sonnenfiltersubstanzen, Antioxidantien und Konservierungsmitteln ausgewählt ist.

6. Zubereitung nach einem der vorstehenden Ansprüche, dadurch gekennzeichnet, daß die wäßrige Phase der Emulsion zusätzlich noch einen Hilfsstoff enthält, der unter Farbstoffen, Konservierungsmitteln, hydrophilen Wirkstoffen, Spurenelementen, biologischen Derivaten, Polyolen, Salzen, Tonmineralien, die in wäßrigem Medium aufquellen, Aminosäuren und wäßrigen Gelierungsmitteln ausgewählt ist.

7. Zubereitung nach einem der vorstehenden Ansprüche, daurch gekennzeichnet, daß sie zusätzlich noch mindestens einen Wirkstoff enthält, der unter essentiellen Ölen, Duftstoffen, Pigmenten, Trägerstoffen, Vitaminen und Ceramiden ausgewählt ist.
